# EUROPEAN PATENT APPLICATION

(11) **EP 2 502 643 A1**
(43) Date of publication of application: **26.09.2012**
(21) Application number: 11159102.0
(22) Date of filing: 22.03.2011
(51) Int. Cl.: A61M 5/24, A61M 5/31, A61M 5/315

(54) **Drug delivery device with pack size reduction**

(71) Applicant: Sanofi-Aventis Deutschland GmbH, 65929 Frankfurt (DE)
(72) Inventor: Jugl, Michael, 65926 Frankfurt am Main (DE); Teucher, Axel, 65926 Frankfurt am Main (DE)

(57) **Abstract**

The present invention relates to a drug delivery device for dispensing a dose of a medicament comprising:
- a first housing component (12), and
- a second housing component (14) longitudinally arranged with the first housing component (12) in an operation mode (50), and
- a size reduction mechanism (32,34) for transferring first and second housing components (12,14) relative to each other into a compact storage configuration (44).

## Description

The present invention relates to a drug delivery device adapted to administer a dose of a medicament by way of injection. In particular, the invention refers to injection devices, such like pen-type injectors adapted to dispense a predefined dose of a medicament.

### Background and Prior Art

Drug delivery devices for setting and dispensing multiple doses of a liquid medicament are as such well-known in the art. Generally, such devices have substantially a similar purpose as that of an ordinary syringe.

Drug delivery devices, in particular pen-type injectors have to meet a number of user specific requirements. For instance, with patients suffering a chronic disease e.g. diabetes, the patient may be physically infirm and may also have impaired vision. Therefore, suitable drug delivery devices need to be robust in construction and should be easy to use. Furthermore, manipulation and general handling of the device and its components should be intelligible and understandable. Moreover, a dose setting and a dose dispensing procedure must be easy to operate and unambiguous. When the device is of disposable type, it should be inexpensive to manufacture and easy to dispose. In order to meet these requirements, the number of parts and steps required to utilize the device should be kept to a minimum.

Typically, drug delivery devices such as pen-type injectors feature a pen-like and rather longitudinal housing. In a distal housing portion, facing towards the patient during a dose dispensing procedure, there is typically arranged a cartridge comprising a cylindrical barrel filled with a medicament and sealed with a displaceable piston. By exerting pressure in distal direction to the piston in a controlled way, a pre-defined amount of a liquid medicament can be expelled from the cartridge. A piston rod to be operably engaged with the piston is typically driven by a drive mechanism positioned in a proximal housing portion of the drug delivery device.

The axial dimension or longitudinal extension of the cartridge and a displacement path of the piston disposed therein require respective axial and longitudinal dimensions of the drive mechanism and its piston rod.

Therefore, drug delivery devices of pen-injector type are typically quite elongated and require a rather longish packaging. Moreover, such devices require a corresponding, quite elongated storage space. In particular, when the device is to be kept in a refrigerated environment, generally suitable refrigerating devices may fail to provide a corresponding rather longish or spacious storage capacity.

### Objects of the Invention

It is therefore an aim of the present invention to provide a drug delivery device, in particular a pen-type injector, having a compact design and requiring reduced, less elongated but compacted storage space. Moreover, the drug delivery device should be easy and unambiguous to handle, both in terms of setting and and dispensing of a dose as well as in terms of storing the device in a compact way.

### Summary of the Invention

The present invention provides a drug delivery device for dispensing a dose of a medicament. The drug delivery device comprises at least two housing components, namely a first housing component and a second housing component, which in an operation mode of the drug delivery device are longitudinally arranged with respect to each other. When first and second housing components comprise a rather cylindrical and elongated shape, first and second housing components are aligned along a common cylinder axis. The resulting geometry of the drug delivery device in operation mode is rather elongated, it is e.g. of cylindrical shape.

In order to transfer the drug delivery device into a compact storage configuration, a size reduction mechanism is provided allowing to transfer or to move first and second housing components relative to each other into a compact storage configuration. Hence, in the storage configuration, first and second housing components are no longer aligned in an elongated and longitudinal way but may be arranged next to each other. This way and by virtue of the size reduction mechanism, the longitudinal extension of the drug delivery device can be effectively reduced and may be in the range of the axial extension of the first and/or the second housing component.

According to a preferred embodiment, first and second housing components are interconnected with each other by way of the size reduction mechanism. In operation mode as well as in the storage configuration, first and second housing components are mechanically coupled with respect to each other. The size reduction mechanism serves to modify the mutual arrangement of first and second housing components without separating them from each other. By avoiding a separation of first and second housing components, the size reduction mechanism also provides an effective anti-loss mechanism.

According to a further embodiment, first and second housing components even remain interconnected during a transfer from operation mode to storage configuration, and vice versa. Preferably, the size reduction mechanism allows to move first and second housing components relative to each other and prevents or impedes mutual separation of said housing components.

According to a further embodiment, first and second housing components are locked in storage configuration and/or in operation mode by means of at least one interlock member. Preferably, the device comprises at least two interlock members, each of which being adapted to interlock the device in operation mode and in storage configuration, respectively. This way, unintended relative movement of first and second housing components can be effectively prevented.

In a further preferred embodiment, the size reduction mechanism comprises a hinge interconnecting first and second housing components with each other. By way of the hinge, typically arranged at a lateral side section of first and/or second housing components, said housing components can be folded and unfolded with respect to each other. For instance, the second housing component may be folded by up to 180° between a storage configuration and an operation mode. This way, first and second housing components may be arranged next to each other, in lateral direction to end up in the storage configuration. In operation mode, they may be co-aligned in longitudinal direction in such a way, that a piston rod of the drive mechanism is enabled to exert distally directed pressure onto a piston of a cartridge.

In a further embodiment, the interlock member comprises a snap-fit connection arranged opposite the hinge, connecting first and second housing components. By means of a snap-fit connection, a releasable and intuitive interlocking of first and second housing components can be provided. The snap-fit connection may automatically interlock as soon as first and second housing components become longitudinally aligned and reach a relative position that corresponds to their operation mode. For releasing the snap-fit connection, a simple depression of a respective positive-engaging snap-fit member may suffice.

In a similar way, first and second housing components may be mutually immobilized when in storage configuration. For instance, first and second housing components may comprise inter-engaging and mutually corresponding locking members at a side section or at end sections, in which first and second housing components mutually get in contact when reaching the storage configuration.

In an alternative embodiment, the size reduction mechanism comprises at least one sliding guide for slidably displacing first and second housing components relative to each other. This way, mutual displacement of first and second housing components may comprise a longitudinally and/or transversely directed translational displacement.

Preferably, the sliding guide extends in longitudinal direction and/or in transverse direction with respect to the geometry of the first and/or the second housing components. Also, when implemented as a sliding mechanism, the size reduction mechanism may be equipped with some kind of interlock means, e.g. a releasable, inter-engaging snap-fit connection.

It is of further benefit and according to another preferred embodiment, when the first housing component comprises a drive mechanism having a piston rod to be operably engaged with a piston of a cartridge disposed in the second housing component. Therefore, when transferring or moving first and second housing components relative to each other, e.g. into the storage configuration, mechanical engagement of the piston rod and the piston of the cartridge is at least temporally abrogated. However, when first and second housing component return into the operation mode, mechanical interaction of the piston and piston rod is rebuilt or reestablished.

By separating the housing of the drug delivery device into a drive mechanism housing component and into a cartridge holder component, replacement of an empty cartridge can be supported and facilitated. It is even conceivable, that once the device is in storage configuration, an empty cartridge can be replaced by a filled or new one. In this context, it is even of further benefit, when the cartridge is fixed in the second housing component by any kind of fixing means, thus preventing an unintentional disassembling of cartridge and second housing component, when the device is in storage configuration.

In a further preferred embodiment, the drive mechanism is adapted to control and/or to initiate a distally directed longitudinal motion of the piston rod. Preferably, the drive mechanism is coupled with the size reduction mechanism. This way, the piston rod of the drive mechanism can be operably connected with the piston of the cartridge as soon as first and second housing components are transferred in or reach the operation mode. It is of particular benefit, when the drive mechanism is electric-mechanically implemented, so as to establish a mechanical coupling of piston and piston rod as soon as the housing components are positioned in operation mode.

In still another embodiment, the drive mechanism is further adapted to transfer the piston rod in a retracted storage position in response to a transfer of first and second housing components into their storage configuration. Depending on the filling level of the cartridge and a respective axial position of the cartridge's piston, the piston rod may protrude in axial and distal direction when in operation mode. In order to allow a relative displacement of cartridge and piston rod, an initial retraction of the piston rod in proximal direction may be required prior to move or to fold first and second housing components relative to each other.

Typically and according to a further embodiment, first and second housing components at least in sections mutually abut or get in contact along a lateral surface portion when they reach their storage configuration. This way, a rather robust geometry of first and second housing components can be attained, which is beneficial for storage and transport of the drug delivery device.

In a further preferred aspect, the cartridge disposed in the drug delivery device is at least partially filled or pre-filled with a medicament, like insulin or heparin. The cartridge may be replaceable when empty but in alternative embodiments, the drug delivery device may be also designed as disposable device intended to be discarded in its entirety after consumption of the medicament.

Moreover, the invention further relates to a method of storing or transporting a drug delivery device being adapted for dispensing a dose of a medicament. The device has a first housing component and a second housing component which are intended to be longitudinally arranged with respect to each other in an operation mode, in which the device is ready to dispense a pre-defined amount of a liquid medicament. The drug delivery device can be transferred into a compact storage configuration by pivoting and/or sliding first and second housing components relative to each other.

A typical scenario for transferring the drug delivery device into a storage configuration may comprise an initial unlocking or releasing of an interlock means generally intended to keep first and second housing components in an operating mode. After releasing or unlocking first and second housing components, the components are free to move and/or to pivot with respect to each other. If the two housing components are for instance interconnected by means of a hinge, the second housing component, preferably in form of a cartridge holder section, can be pivoted by about 180° such that first and second housing components become arranged next to each other, i.e. in radial direction with respect to the long axis or symmetry axis of the housing components.

In this folded configuration, first and second housing components may become subject to a further locking procedure in order to keep the drug delivery device in the compact storage configuration.

For re-establishing of an operation mode, first and second housing components have to be released initially and the second housing component has to be pivoted back into its operation configuration, in which first and second housing components are to be interlocked again. Finally, prior to a subsequent dose setting and dispensing, the drive mechanism of the drug delivery device may perform an automated set-up procedure for bringing piston and piston rod into a mutual mechanical contact or abutment.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

### Pharmaceutically acceptable solvates are for example hydrates.

It will be further apparent to those skilled in the pertinent art that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following, preferred embodiments of the invention will be describe in greater detail by making reference to the drawings in which:
- Figure 1: illustrates a drug delivery device in form of a pen-type injector in a perspective view,
- Figure 2: shows a schematic illustration of a pen-type injector in a folded storage configuration,
- Figure 3: shows the pen-type injector of Figure 2 during transformation towards an operation mode,
- Figure 4: illustrates a further pivoting of the housing components of the drug delivery device of Figure 3, and
- Figure 5: shows the drug delivery device in operation mode,
- Figure 6: shows another size reduction mechanism of a drug delivery device in storage configuration,
- Figure 7: shows the drug delivery device according to Figure 6 with a second housing component axially shifted,
- Figure 8: shows the drug delivery device according to Figures 6 and 7 with a further shifted configuration of first and second housing components, and
- Figure 9: illustrates the drug delivery device according to Figures 6 to 8 in operation mode, and
- Figure 10: illustrates a sliding guide mechanism of the two housing components along a cross section A-A according to Figure 7.

### Detailed Description

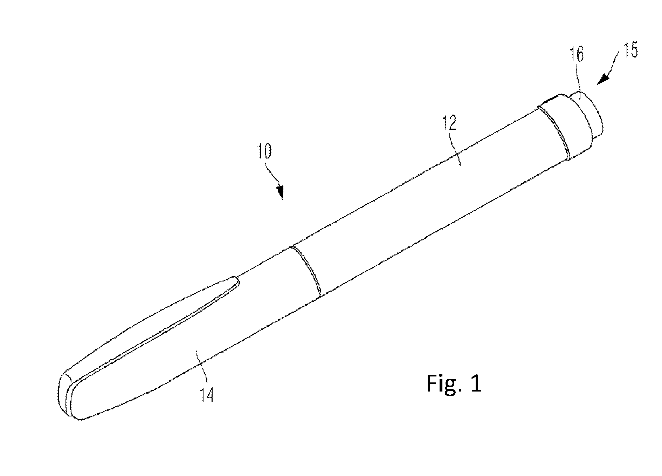

Figure 1 schematically illustrates a drug delivery device 10 designed as a pen-type injector. The device 10 comprises a proximal housing component 12 as well as a distal housing component 14. The distal housing component 14 may act as a protective cap or as a cartridge holder to keep a cartridge 18 filled with a medicament in position. At a proximal end 15, the drug delivery device comprises a dose button 16 by way of which a dose to be dispensed by the device 10 can be individually selected and dispensed. The distal end 17 of the drug delivery device 10 and in particular the distal section of a cartridge holder is adapted to be releasably coupled with a piercing assembly, such like an injection needle, in order to dispense a pre-defined amount of the liquid medicament by way of injection.

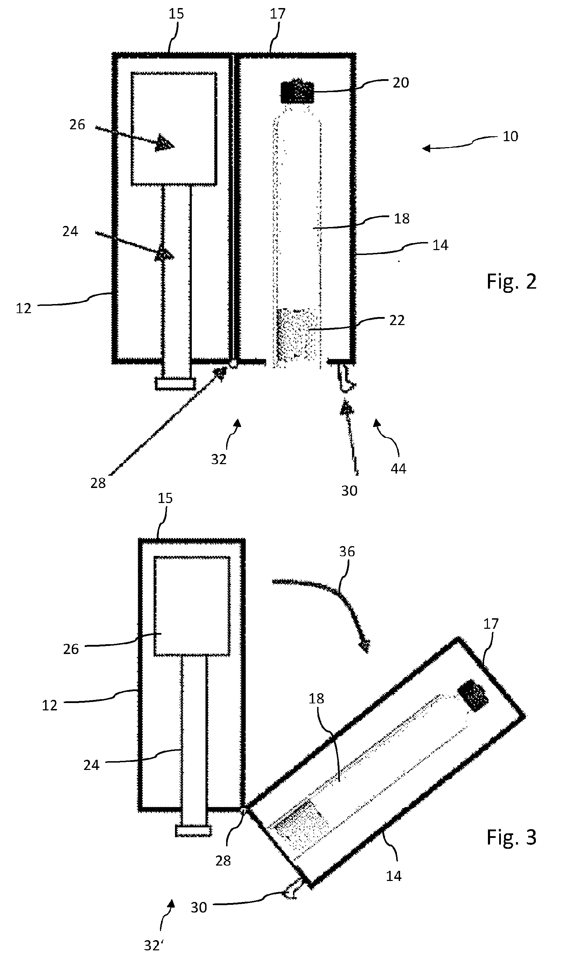

The sequence according to Figures 2 to 5 illustrates a first size reduction mechanism 32 allowing to reduce the longitudinal extension of the drug delivery device 10. As illustrated in Figure 2, cartridge holder or cap section 14 is pivotally connected with a proximal housing section 12 adapted to house a drive mechanism 26 together with a piston rod 24. A hinge 28 interconnecting the proximal 12 and the distal housing component 14, allows to pivot first and second housing components 12, 14 with respect to each other by about 180°.

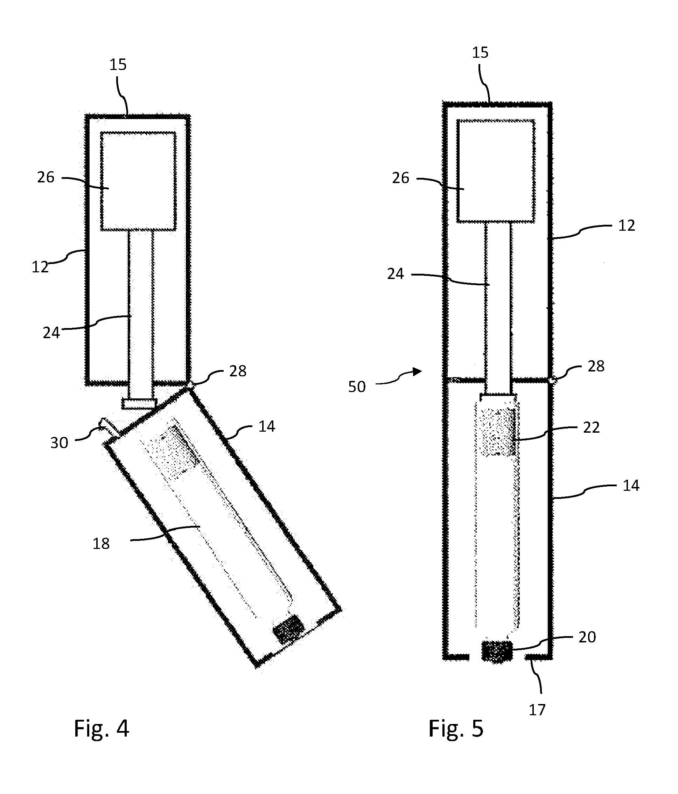

In the sequence of Figures 3, 4 and 5, such a pivoting motion is stepwise illustrated by depicting the stepwise modified size reduction mechanism 32'. Upon reaching the assembled end configuration as depicted in Figure 5, the piston rod 24 gets in direct mechanical contact with the piston 22 of the cartridge 18. By applying distally, hence downwardly directed pressure towards the piston 22, a pre-defined amount of the liquid medicament contained therein can be expelled via the distal seal 20 of the cartridge 18, which is typically designed as a pierceable septum.

Moreover, in order to keep and to attain the operation mode 50 as illustrated in Figure 5, the distal or second housing component 14 is further equipped with an interlock member 30 adapted to positively engage with a corresponding side wall section of the proximal and first housing component 12. Typically, the interlock member 30 is designed as a snap-fit connection allowing to release the interlocking on demand. This way, the second housing component 14 may return into its storage configuration 44 as depicted in Figure 2, e.g. in which the device no longer requires a longish but only a rather compact storage space. In an alternative, it is conceivable, that the interlock member 30 is arranged at the proximal housing component 12, or that both housing components 12, 14 comprise mutually corresponding interlock members 30.

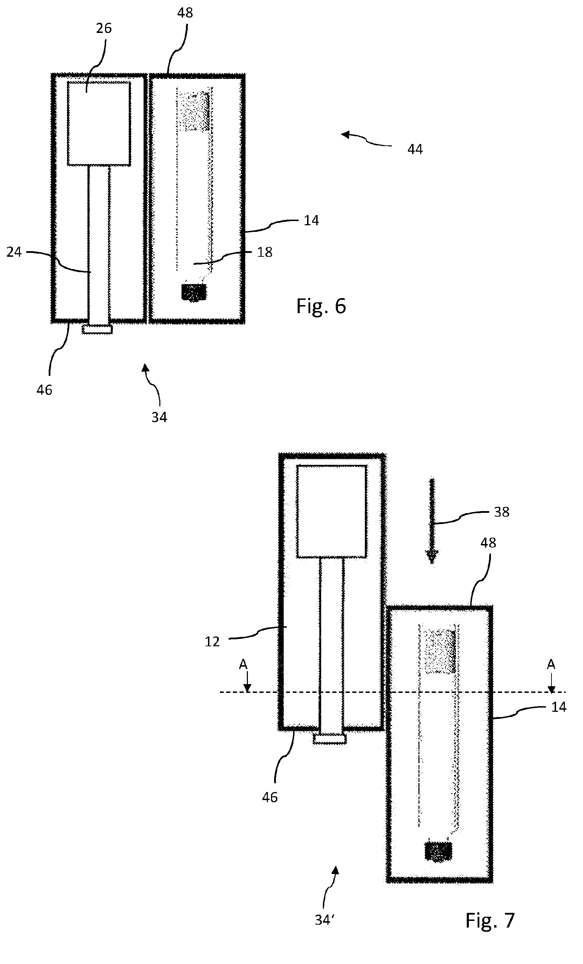

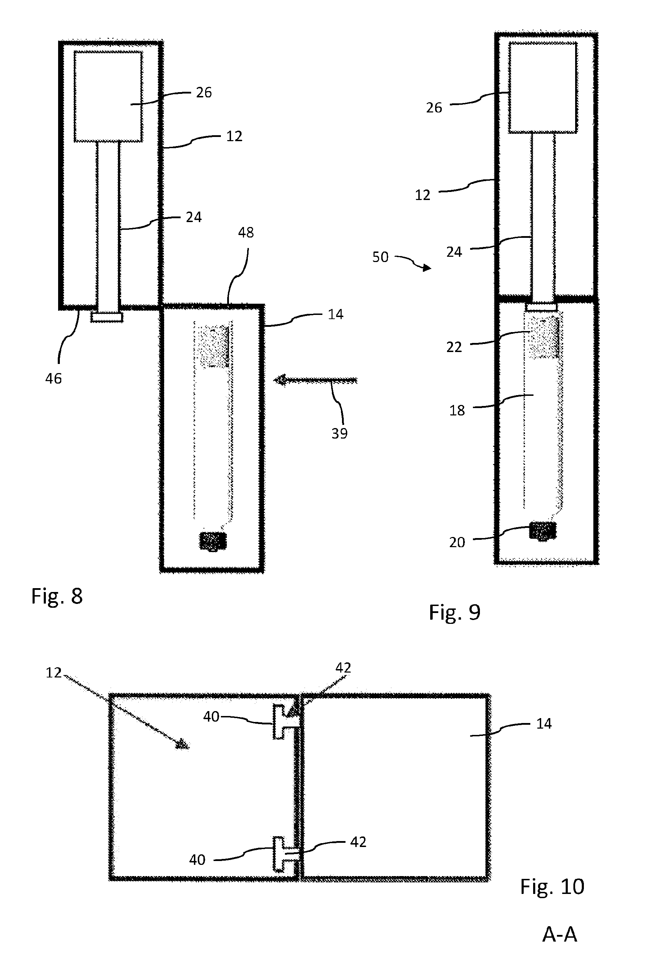

In the sequence of Figures 6 to 9, another embodiment of a size reduction mechanism 34 is illustrated. Also here, first and second housing components 12, 14 are arranged next to each other in a storage configuration 44 as depicted in Figure 6. However, relative motion of first and second housing components 12, 14 is substantially based on a sliding and translational movement of the second housing component 14 relative to the first housing component 12. As indicated in Figure 7, the second housing component 14 can be moved in distal direction 38 relative to the first housing component 12 by a sliding motion until it reaches a distal end section of the first housing component 12, as depicted in Figure 8.

Once the two housing components 12, 14 do no longer overlap in radial direction, hence in horizontal direction according to Figure 8, the distal and second housing component 14 may be moved radially inwardly as indicated by arrow 39 relative to the first or proximal housing component 12. In this intermediate configuration 34 of the size reduction mechanism, another sliding guide mechanism may take action allowing for a relative sliding displacement in radial or transverse direction until first and second housing components 12, 14 reach an operation mode 50 as depicted in Figure 9, in which first and second housing components 12, 14 are aligned to each other. Also here, first and second housing components 12, 14 may be interlocked by way of some kind of interlock mechanism, which, in this case not explicitly illustrated.

Figure 10 finally illustrates an example of a sliding guide to move first and second housing components 12, 14 relative to each other. The sketch of Figure 10 showing a cross section A-A according to Figure 7 is illustrated without the piston rod 24 and the corresponding cartridge 18.

As illustrated there, the first housing component 12 comprises two laterally separated substantially T-shaped guiding grooves 40, each of which being adapted to receive a correspondingly T-shaped peg 42 laterally protruding from the second housing component 14. In a similar way, also a distal end face 46 of the first housing component 15 as well as a proximal end face 48 of the second housing component 14 may be equipped with mutually corresponding positively engaging sliding or guiding members in order to provide a controlled lateral displacement of first and second housing components 12, 14 as indicated in Figure 8.

### List of Reference Numerals

- 10: drug delivery device
- 12: housing component
- 14: housing component
- 15: proximal end section
- 16: dose button
- 17: distal end section
- 18: cartridge
- 20: seal
- 22: piston
- 24: piston rod
- 26: drive mechanism
- 28: hinge
- 30: interlock member
- 32: reduction mechanism
- 34: reduction mechanism
- 36: pivoting motion
- 38: distal direction
- 39: transverse direction
- 40: groove
- 42: peg
- 44: storage configuration
- 46: distal end face
- 48: proximal end face
- 50: operation mode

## Claims

1. Drug delivery for dispensing a dose of a medicament comprising:
- a first housing component (12), and
- a second housing component (14) longitudinally arranged with the first housing component (12) in an operation mode (50), and
- a size reduction mechanism (32, 34) for transferring first and second housing components (12, 14) relative to each other into a compact storage configuration (44).

2. The drug delivery device according to claim 1, wherein first and second housing components (12, 14) are interconnected with each other in operation mode (50) and in the storage configuration (44).

3. The drug delivery device according to any one of the preceding claims,
wherein first and second housing components (12, 14) remain interconnected during a transfer from the operation mode (50) into the storage configuration (44), and vice versa.

4. The drug delivery device according to any one of the preceding claims,
wherein first and second housing components (12, 14) are locked in storage configuration (44) and/or in operation mode (50) by means of at least one interlock member (30).

5. The drug delivery device according to any one of the preceding claims,
wherein the size reduction mechanism (32) comprises a hinge (28) interconnecting first and second housing components (12, 14).

6. The drug delivery device according to claim 5, wherein the interlock member (30) comprises a snap-fit connection arranged opposite the hinge (28).

7. The drug delivery device according to any one of the preceding claims 1 to 4,
wherein the size reduction mechanism (34) comprises at least one sliding guide (40; 42) for slidably displacing first and second housing components (12, 14) relative to each other.

8. The drug delivery device according to claim 7, wherein the sliding guide (40, 42) extends in longitudinal direction (38) and/or in transverse direction (39) with respect to the geometry of the first and/or the second housing components (12, 14).

9. The drug delivery device according to any one of the preceding claims, wherein the first housing component (12) comprises a drive mechanism (26) having a piston rod (24) to be operably engaged with a piston (22) of a cartridge (18) disposed in the second housing component (14).

10. The drug delivery device according to any one of the preceding claims, wherein the drive mechanism (26) is coupled with the size reduction mechanism (32, 34) for operably connecting the piston rod (24) with the piston (22) of the cartridge (18) when first and second housing components (12, 14) are transferred in operation mode (50).

11. The drug delivery device according to any one of the preceding claims,
wherein the drive mechanism (26) is adapted to move the piston rod (24) in a retracted storage position in response to a transfer of first and second housing components (12, 14) into their storage configuration (44).

12. The drug delivery device according to any one of the preceding claims,
wherein the first and second housing components (12, 14) at least in sections mutually abut along a lateral surface when in storage configuration (44).

13. The drug delivery device according to any one of the preceding claims, further comprising a cartridge (18) being at least partially filled with a medicament.

14. A method of storing and/or transporting a drug delivery device adapted to dispense a dose of a medicament, the device having a first housing component (12), and a second housing component (14) longitudinally arranged with the first housing component (12) in an operation mode (50),
wherein the drug delivery device is transferred into a compact storage configuration (44) by pivoting and/or sliding first and second housing components (12, 14) relative to each other.
